# NEW EUROPEAN PATENT SPECIFICATION

(11) **EP 2 091 507 B2**
(45) Date of publication and mention of the opposition decision: **01.08.2018**
(45) Mention of the grant of the patent: 25.03.2015
(21) Application number: 07822287.4
(22) Date of filing: 07.11.2007
(51) Int. Cl.: A61K 8/34, A61K 8/41, A61K 8/42, A61K 8/73, A61Q 5/02, A61Q 5/12

(54) **HAIR CONDITIONING COMPOSITIONS**
HAARKONDITIONIERUNGSZUSAMMENSETZUNGEN
COMPOSITIONS APRES-SHAMPOOING

(30) Priority: 02.12.2006 GB 0624132
(43) Date of publication of application: 26.08.2009
(73) Proprietor: Unilever PLC, London EC4Y 0DY (GB); Unilever N.V., 3013 AL Rotterdam (NL)
(72) Inventor: EVERAERT, Emmanuel Paul Jos Marie, Bebington, Wirral Merseyside CH63 3JW (GB); MURRAY, Andrew Malcolm, Bebington, Wirral Merseyside CH63 3JW (GB); PHAM, Thuy-Anh, Bebington, Wirral Merseyside CH63 3JW (GB); PUNTAMBEKAR, Smita, Bebington, Wirral Merseyside CH63 3JW (GB)
(74) Representative: Chisem, Janet
(86) International application number: PCT/EP2007/061971
(87) International publication number: WO 2008/064976

(56) References cited:
- EP-A- 0 035 899
- EP-A- 1 800 658
- WO-A1-2006/093714
- US-A- 4 870 167
- US-A- 6 132 707
- US-A1- 2005 226 842
- US-A1- 2006 210 509
- US-B1- 6 387 855

## Description

### FIELD OF THE INVENTION

The present invention relates to hair conditioning compositions which comprise a polygalactomannan having hydrophilic and hydrophobic substituents.

### BACKGROUND TO THE INVENTION AND PRIOR ART

Hair conditioning compositions are typically applied to the hair immediately after shampooing and rinsing the hair. The conditioning composition is worked through the hair, and may then be left to penetrate the hair for a period of time before rinsing it from the hair with water.

Traditionally such conditioning compositions have used a combination of cationic surfactants and fatty materials such as long chain fatty alcohols. This combination forms a lamellar gel phase which imparts a desirable viscosity to the product and deposits on the hair during use of the product to provide a conditioning benefit.

Many consumers desire a "lighter" conditioning product which imparts less of a slippery and coated feel to their hair. This has led to the development of "low-fat" formulations with a reduced content of fatty material.

However, reducing the content of fatty material can also reduce the viscosity of the product to an unacceptable level. Consequently, it has been found to be necessary to incorporate a thickener.

Examples of thickeners which have been used for this purpose are nonionic cellulose ethers such as hydroxyethylcellulose.

Hydrophobically-modified cellulose ethers such as cetyl hydroxyethylcellulose have also been used. Materials of this type are described in EP 412 705, EP 412 706 and EP 412 710 as providing a rheology very much like the gel-network structure of typical hair conditioners without the slimy feel associated with most polymeric thickeners, and without using a typical quaternary ammonium compound/fatty alcohol gel-network thickening system.

A problem associated with the use of the above-described cellulosic thickeners is that it is difficult to obtain the right viscosity profile under different conditions of product usage. For example, a thick, creamy product viscosity is desirable to enable controlled pouring and product dosage onto the hair. A lower product viscosity is preferred to facilitate spreading of the product through the hair and rinsing, but not to the extent that the product is perceived to "disappear" into the hair.

The inventors have found that this problem can be solved if a polygalactomannan having hydrophilic and hydrophobic substituents is used to thicken the hair conditioning composition.

Substituted polygalactomannans of the above type have been proposed for the stabilisation of lathering compositions such as shower gels and shampoos in WO99/01105. The formulations in that publication are based on insoluble silicones and detergent surfactants.

US2006/0210509 describes silicone-based hair conditioning compositions containing cationic surfactants and a nonionic alkoxylated urethane.

EP 1 800 658 A1 is prior art under Art.54(3) EPC. This document discloses a styling composition comprising 1% wt. Styleze W20 (terpolymer of vinylpyrrolidone/dimethylaminopropylmethacrylamide and of lauryldimethyl-methacrylamidopropylammonium chloride), 3wt.% cetyl alcohol, 1.5% wt. DC939 (amodimethicone, trimethylcetylammonium chloride and nonionic surfactant of formula C₁₃H₂₇-(OC₂H₄)₁₂-OH), 1%wt. cetyltrimethylammonium chloride, 0.5% wt. hydroxypropyl guar, 1%wt. glycerol stearate, 2%wt. polyvinyl pyrrolidone, preservatives and to 100% wt. water. This specific disclosure has been disclaimed.

### SUMMARY OF THE INVENTION

The invention provides a hair conditioning composition comprising a cationic surfactant, a fatty material, a polygalactomannan having hydrophilic and hydrophobic substituents, and an aqueous carrier, according to claim 1.

### DETAILED DESCRIPTION OF THE INVENTION

### Cationic Surfactant

Compositions according to the invention comprise one or more cationic surfactants which are cosmetically acceptable and suitable for topical application to the hair.

Suitable cationic surfactants for use in compositions of the invention contain amino or quaternary ammonium hydrophilic moieties which are positively charged when dissolved in the composition.

Suitable quaternary ammonium cationic surfactants correspond to the following general formula (I):

[N(R¹)(R²)(R³)(R⁴)]⁺(X)⁻ (I)

in which R¹, R², R³, and R⁴ are each independently selected from (a) an aliphatic group of from 1 to 22 carbon atoms, or (b) an aromatic, alkoxy, polyoxyalkylene, alkylamido, hydroxyalkyl, aryl or alkylaryl group having up to 22 carbon atoms; and X is a salt-forming anion such as those selected from halogen, (e.g. chloride, bromide), acetate, citrate, lactate, glycolate, phosphate nitrate, sulphate, and alkylsulphate radicals.

The aliphatic groups can contain, in addition to carbon and hydrogen atoms, ether linkages, and other groups such as amino groups. The longer chain aliphatic groups, e.g., those of about 12 carbons, or higher, can be saturated or unsaturated.

In a suitable class of cationic surfactant of general formula (I), R¹ and R² are each independently selected from C₁₆ to C₂₂ hydrocarbyl chains comprising at least one ester linkage in both R¹ and R², and R³ and R⁴ are each independently selected from CH₃ and CH₂CH₂OH.

In another suitable class of cationic surfactant of general formula (I), R¹ and R¹ are each independently selected from C₁₆ to C₂₂ saturated or unsaturated, preferably saturated, chains, and R³ and R⁴ are each independently selected from CH₃ and CH₂CH₂OH, preferably CH₃.

In a preferred class of cationic surfactant of general formula (I), R¹ is a C₁₆ to C₂₂ alkyl chain and R², R³ and R⁴ are each independently selected from CH₃ and CH₂CH₂OH, preferably CH₃.

Specific examples of suitable quaternary ammonium cationic surfactants of general formula (I) are cetyltrimethylammonium chloride, behenyltrimethylammonium chloride (BTAC), cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, dipalmitoylethyldimethylammonium chloride, PEG-2 oleylammonium chloride and salts of these, where the chloride is replaced by halogen, (e.g., bromide), acetate, citrate, lactate, glycolate, phosphate nitrate, sulphate, or alkylsulphate.

Particularly preferred quaternary ammonium cationic surfactants for use in the invention are cetyltrimethylammonium chloride, available commercially, for example as GENAMIN CTAC, ex Hoechst Celanese and Arquad 16/29 supplied by Akzo Nobel, and behenyltrimethylammonium chloride (BTAC) such as Genamin KDM-P supplied by Clariant.

Mixtures of any of the foregoing materials may also be suitable.

Salts of primary, secondary, and tertiary fatty amines are also suitable cationic surfactants for use in the invention. The alkyl groups of such amines preferably have from about 12 to about 22 carbon atoms, and can be substituted or unsubstituted. These amines are typically used in combination with an acid to provide the cationic species.

A preferred class of amine corresponds to the following general formula (II):

R¹-C(O)-N(H)-R²-N(R³)(R⁴) (II)

in which R¹ is a fatty acid chain containing from 12 to 22 carbon atoms, R² is an alkylene group containing from one to four carbon atoms, and R³ and R⁴ are, independently, an alkyl group having from one to four carbon atoms.

Specific examples of suitable materials of general formula (II) are stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, and diethylaminoethylstearamide.

Also useful are dimethylstearamine, dimethylsoyamine, soyamine, myristylamine, tridecylamine, ethylstearylamine, N-tallowpropane diamine, ethoxylated (with 5 moles of ethylene oxide) stearylamine, dihydroxyethylstearylamine, and arachidyl behenylamine.

Particularly preferred is stearamidopropyldimethylamine.

Mixtures of any of the foregoing materials may also be suitable.

The acid used to provide the cationic species can be any organic acid or mineral acid of sufficient acid strength to neutralise a free amine nitrogen. Such acids include hydrochloric acid, sulphuric acid, nitric acid, phosphoric acid, lactic acid, citric acid, tartaric acid, acetic acid, gluconic acid, glycolic acid and propionic acid, or combinations thereof. In general, a sufficient amount of acid is added to neutralise the amidoamine compound and to adjust the final pH of the composition to within a range of from about 2.5 to about 6, preferably in a pH range of from about 3 to about 5. The molar ratio of protonatable amine groups to H⁺ from the acid is preferably from about 1:0.3 to 1:1.2, and more preferably from about 1:0.5 to about 1:1.1. Mixtures of any of the above-described cationic surfactants may also be suitable.

In the composition of the invention, the level of cationic surfactant preferably ranges from 0.1 to 10%, more preferably 0.2 to 5%, most preferably 0.25 to 4% by total weight of cationic surfactant based on the total weight of the composition.

### Fatty Material

Compositions of the invention comprise a fatty material. The fatty material, together with the cationic surfactant and an aqueous carrier, forms a lamellar gel phase which is suitable for providing various hair conditioning attributes. The fatty material is selected from cetyl alcohol, stearyl alcohol, behenyl alcohol, and mixtures thereof.

The level of fatty material in conditioners of the invention suitably ranges from 0.01 to 15%, preferably from 0.1 to 10%, and more preferably from 0.1 to 5% by total weight fatty material based on the total weight of the composition.

The weight ratio of cationic surfactant to fatty material is suitably from 10:1 to 1:10, preferably from 4:1 to 1:8, more preferably from 1:1 to 1:7.

### Substituted Polygalactomannan

Polygalactomannans are polysaccharides composed principally of galactose and mannose units and are usually found in the endosperm of certain leguminous seeds such as guar, locust bean, honey locust, flame tree and the like. Guar gum, for example, is composed mostly of a galactomannan with essentially is a straight chain mannan with single membered galactose branches. The ratio of galactose to mannose in the guar polymer is 1:2. Locust bean gum is a polygalactomannan gum of similar molecular structure in which the ratio of galactose to mannose is 1:4.

Guar and locust bean gums are preferred sources of the polygalactomannans, principally because of their commercial availability. Guar gum is a most preferred source.

Polygalactomannans for use in compositions of the invention (hereinafter referred to as "substituted polygalactomannans") contain hydrophilic substituents and hydrophobic substituents.

Suitably the substituted polygalactomannan is derived from a polygalactomannan having a molecular weight of from 50,000 to 1,600,000, depending on the origin of the polygalactomannan.

Suitable substituted polygalactomannans have a total molar substitution of greater than 0.7. Preferably the total molar substitution ranges from 0.9 to 2.01. By "molar substitution" is meant the average number of moles of substituents on each anhydroglycosidic unit of the polygalactomannan.

Suitable substituted polygalactomannans contain an average of from 0.7 to 4 hydrophilic substituents per anhydroglycosidic unit. Preferred substituted polygalactomannans contain an average of from 0.9 to 2 hydrophilic substituents per anhydroglycosidic unit.

Suitable substituted polygalactomannans contain an average of from 0.0001 to 0.02 hydrophobic substituents per anhydroglycosidic unit. Preferred substituted polygalactomannans contain an average of from 0.0005 to 0.01 hydrophobic substituents per anhydroglycosidic unit.

Suitably the molar ratio of hydrophilic substituents to hydrophobic substituents ranges from 35:1 to 40,000:1. Preferably the molar ratio ranges from 90:1 to 4000:1.

The hydrophilic substituents may suitably be selected from C₂-C₄ alkyl groups, C₂-C₄ hydroxyalkyl groups, carboxymethyl, amino and carboxylic groups. Preferably the hydrophilic substituents are C₂-C₄ hydroxyalkyl groups, most preferably hydroxypropyl groups. Mixtures of any of the above hydrophilic substituents may also be suitable.

The hydrophobic substituents may suitably be chosen from linear or branched alkyl and alkenyl groups containing from 10 to 32 carbon atoms, more preferably from 14 to 28 carbon atoms. The alkyl or alkenyl groups can be substituted with one or more hydroxyl groups. Mixtures of any of the above hydrophobic substituents may also be suitable.

The hydrophilic and hydrophobic substituents can either be linked directly via a carbon-carbon bond to the anhydroglycoside unit, or can be linked via an ether, urethane, ester, amide or acyl bond and preferably via an ether bond.

Preferred substituted polygalactomannans for use in the invention are poly (alkyl ethers) of polygalactomannans having hydrophilic and hydrophobic alkyl ether substituents respectively.

Examples of such materials are poly (alkyl ethers) of polygalactomannans in which the hydrophilic substituent is HOR¹-, in which R¹ is an alkylene group having two to four carbon atoms and in which the OH group is on the carbon atom beta to the ether group, and the hydrophobic substituent is chosen from R², HOR³, and R⁴O-CH₂CH(OH)-CH₂-, in which R² is an alkyl group which contains 10 to 32 carbon atoms, R³ is an alkylene group which contains 10 to 32 carbon atoms having the OH group on the carbon atom beta to the ether group and R⁴ is an alkyl group having from 7 to 29 carbon atoms.

Preferred examples of such materials are poly (alkyl ethers) of polygalactomannans in which the hydrophilic substituent is the hydroxypropyl group and the hydrophobic substituent is a linear alkyl group containing from 14 to 28 carbon atoms or a mixture of such alkyls.

US 4,960,876 and US 4,870,167 describe methods of preparation of substituted polygalactomannans suitable for use in compositions of the invention.

Commercially available examples of substituted polygalactomannans suitable for use in compositions of the invention are guar gums sold under the name Esaflor HM 22 by the company Lamberti or under the name Jaguar XC 95-3 by the company Rhône-Poulenc.

The level of substituted polygalactomannan in conditioners of the invention suitably ranges from 0.001 to 10%, preferably from 0.005 to 5%, and more preferably from 0.01 to 2% by total weight substituted polygalactomannan based on the total weight of the composition.

### Aqueous Carrier

The conditioning composition of the present invention comprises an aqueous carrier.

Suitable aqueous carriers are water and water solutions of lower alkyl alcohols and polyhydric alcohols.

Examples of suitable lower alkyl alcohols are monohydric alcohols having 1 to 6 carbons, preferably ethanol and isopropanol.

Examples of suitable polyhydric alcohols are propylene glycol, hexylene glycol, glycerin, and propanediol.

Preferably, the aqueous carrier is substantially water.

Generally, compositions according to the invention comprise at least 60%, preferably at least 65%, more preferably at least 70% water by weight based on the total weight of the composition.

### Further Conditioning Agents

Compositions of the invention may comprise further conditioning agents to optimise wet and dry conditioning benefits.

Particularly preferred further conditioning agents are silicone emulsions.

Suitable silicone emulsions include those formed from silicones such as polydiorganosiloxanes, in particular polydimethylsiloxanes which have the CTFA designation dimethicone, polydimethyl siloxanes having hydroxyl end groups which have the CTFA designation dimethiconol, and amino-functional polydimethyl siloxanes which have the CTFA designation amodimethicone.

The emulsion droplets may typically have a Sauter mean droplet diameter (D_{3,2}) in the composition of the invention ranging from 0.01 to 20 micrometer, more preferably from 0.2 to 10 micrometer.

A suitable method for measuring the Sauter mean droplet diameter (D_{3,2}) is by laser light scattering using an instrument such as a Malvern Mastersizer.

Suitable silicone emulsions for use in compositions of the invention are available from suppliers of silicones such as Dow Corning and GE Silicones. The use of such pre-formed silicone emulsions is preferred for ease of processing and control of silicone particle size. Such pre-formed silicone emulsions will typically additionally comprise a suitable emulsifier such as an anionic or nonionic emulsifier, or mixture thereof, and may be prepared by a chemical emulsification process such as emulsion polymerisation, or by mechanical emulsification using a high shear mixer. Pre-formed silicone emulsions having a Sauter mean droplet diameter (D_{3,2}) of less than 0.15 micrometers are generally termed microemulsions.

Examples of suitable pre-formed silicone emulsions include emulsions DC2-1766, DC2-1784, DC-1785, DC-1786, DC-1788 and microemulsions DC2-1865 and DC2-1870, all available from Dow Corning. These are all emulsions/microemulsions of dimethiconol. Also suitable are amodimethicone emulsions such as DC939 (from Dow Corning) and SME253 (from GE Silicones).

Also suitable are silicone emulsions in which certain types of surface active block copolymers of a high molecular weight have been blended with the silicone emulsion droplets, as described for example in WO03/094874. In such materials, the silicone emulsion droplets are preferably formed from polydiorganosiloxanes such as those described above. One preferred form of the surface active block copolymer is according to the following formula: 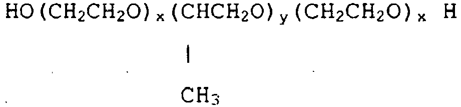 wherein the mean value of x is 4 or more and the mean value of y is 25 or more.

Another preferred form of the surface active block copolymer is according to the following formula: 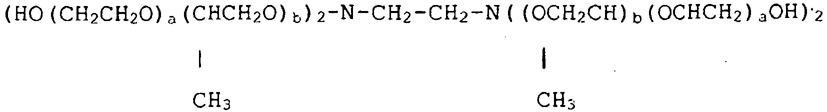 wherein the mean value of a is 2 or more and the mean value of b is 6 or more.

Mixtures of any of the above described silicone emulsions may also be used.

Silicone will generally be present in a composition of the invention at levels of from 0.05 to 10%, preferably 0.05 to 5%, more preferably from 0.5 to 2% by total weight of silicone based on the total weight of the composition.

### Other Optional Ingredients

Compositions according to the invention may also incorporate other cosmetically suitable ingredients, preferably at a level of 2% by weight or less. Suitable ingredients include: preservatives, colouring agents, chelating agents, antioxidants, fragrances, antimicrobials, antidandruff agents, cationic conditioning polymers, styling ingredients, sunscreens, proteins and hydrolysed proteins.

### Use

The compositions of the invention may be used by applying them to wet hair, typically hair which has been shampooed and then rinsed with water.

Generally, the composition is applied to the hair and then worked through the hair. Preferably the composition is then left to penetrate the hair for a period of about one to three minutes before rinsing it from the hair with water.

The invention will now be further described by reference to the following Examples. In the Examples, all percentages are by weight based on total weight, unless otherwise specified. Examples according to the invention are denoted by a number, whereas comparative examples are denoted by a letter.

### EXAMPLES

A series of hair conditioning compositions were prepared having ingredients as shown in the following Table 1:

**Table 1**

| **Ingredient** | **A** | **B** | **1** | **C** | **2** | **D** | **3** |
|---|---|---|---|---|---|---|---|
| Cetyl trimethyl ammonium chloride (29% active) | 2.4 | 2.4 | 2 | - | - | - | - |
| Stearamidopropyl dimethylamine (acid-neutralised) | - | - | - | 0.7 | 0.7 | 0.7 | 0.7 |
| Cetearyl alcohol | 2.1 | 2.1 | 2.1 | - | - | - | - |
| Cetostearyl alcohol | - | - | - | 2.5 | 2.5 | 3 | 3 |
| NATROSOL® 250HHR⁽¹⁾ | 1 | - | - | 1 | - | - | - |
| POLYSURF® 67⁽²⁾ | - | 0.04 | - | - | - | 0.06 | - |
| ESAFLOR® HM22⁽³⁾ | - | | 0.4 | - | 0.4 | - | 0.3 |
| Silicone (60% active) | - | - | - | - | - | 1.7 | 1.7 |
| Phenoxyethanol | 0.4 | 0.4 | 0.4 | | | - | - |
| Methyl Paraben | - | - | - | 0.4 | 0.4 | 0.4 | 0.4 |
| Perfume Water | 0.5 to 100 | 0.5 to 100 | 0.5 to 100 | 0.5 to 100 | 0.5 to 100 | 0.5 to 100 | 0.5 to 100 |
| ⁽¹⁾ Hydroxyethylcellulose, ex Aqualon | | | | | | | |
| ⁽²⁾ Cetyl hydroxyethylcellulose, ex Aqualon | | | | | | | |
| ⁽³⁾ Hydrophobically modified hydroxypropyl guar, ex Lamberti | | | | | | | |

The compositions were evaluated for their viscosity behaviour under varying conditions of shear stress.

The results of the evaluation are shown below in Table 2:

**Table 2**

| **Viscosity (Pa.s)** | **Shear Stress (Pa)** | **A** | **B** | **1** | **C** | **2** | **D** | **3** |
|---|---|---|---|---|---|---|---|---|
| | 0.1 Pa | 632 | 1580 | 1983 | 42 | 988 | 3147 | 4748 |
| | 10 Pa | 148 | 1553 | 2326 | 11 | 883 | 1438 | 4843 |
| | 50 Pa | 1.25 | 0.53 | 311 | 1.5 | 21 | 0.32 | 83.65 |
| | 100 Pa | 0.2 | 0.05 | 0.5 | 0.34 | 0.14 | 0.08 | 0.24 |

The results show that the Examples according to the invention (1, 2 and 3) have a superior viscosity profile under conditions of low and high shear, when compared to Comparative Examples (A, B, C, D) with the same surfactant base.

Compositions according to the invention have a desirable thick viscosity at low shear, which does not break down too rapidly at high shear. If viscosity breakdown is too rapid at high shear (for example as shown in Comparative Example B), the conditioner will be perceived to "disappear" into the hair when it is spread through the hair by the consumer.

## Claims

1. A hair conditioning composition comprising a cationic surfactants, a fatty material, a polygalactomannan having hydrophilic and hydrophobic substituents, and an aqueous carrier, wherein the composition is not a styling compo-sition comprising 1% wt. Styleze W20 (terpolymer of vinylpyrrolidone/dimethylaminopropyl methacrylamide and of lauryldimethylmethacrylamidopropylammonium chloride,, 3% wt. cetyl alcohol, 1.5% wt. DC 939 (amodimethicone, trimethylcetylammonium chloride, and nonionic surfactant of formula C13H27-(OC2H4)12-OH), 1% wt. cetyltrimeth-ylammonium chloride, 6.5% wt. hydroxypropyl guar, 1% wt. glycerol stearate, 2% wt. polyvinyl pyrrolidone, preserv-atives and to 100% wt. water, wherein the fatty material is selected from cetyl alcohol, stearyl alcohol, behenyl alcohol and mixtures thereof, and wherein the fatty material together with the cationic surfactant and the aqueous carrier, forms a lamellar gel phase.

2. A hair conditioning composition according to claim 1, in which the cationic surfactant is a quaternary ammonium cationic surfactant corresponding to the following general formula (I):
[N(R1)(R2)(R3)(R4)]+(X)- (I)
in which R1, R2, R3, and R4 are each independently selected from (a) an aliphatic group of from 1 to 22 carbon atoms, or (b) an aromatic, alkoxy, polyoxyalkylene, alkylamido, hydroxyalkyl, aryl or alkylaryl group having up to 22 carbon atoms; and X is a salt-forming anion such as those selected from halogen, (e.g. chloride, bromide), acetate, citrate, lactate, glycolate, phosphate nitrate, sulphate, and alkylsulphate radicals.

3. A hair conditioning composition according to claim 1, in which the cationic surfactant is a salt of an amine corresponding to the following general formula (II):
R1-C(O)-N(H)-R2-N(R3)(R4) (II)
in which R1 is a fatty acid chain containing from 12 to 22 carbon atoms, R2 is an alkylene group containing from one to four carbon atoms, and R3 and R4 are, independently, an alkyl group having from one to four carbon atoms.

4. A hair conditioning composition according to any one of claims 1 to 3, in which the polygalactomannan having hydrophilic and hydrophobic substituents is a poly (alkyl ether) of a polygalactomannan having hydrophilic and hydrophobic alkyl ether substituents respectively.

5. A hair conditioning composition according to claim 4, in which the hydrophilic substituent is the hydroxypropyl group and the hydrophobic substituent is a linear alkyl group containing from 14 to 28 carbon atoms or a mixture of such alkyls.

## Patentansprüche

1. Zusammensetzung zum Konditionieren der Haare,
die ein kationisches Tensid, ein Fettmaterial, ein Polygalactomannan mit hydrophilen und hydrophoben Substituenten und einen wässrigen Träger aufweist, wobei die Zusammensetzung keine Stylingzusammensetzung ist, die 1 Gew.-% Styleze W20 (Terpolymer von Vinylpyrrolidon/Dimethylaminopropylmethacrylamid und Lauryldimethylmethacrylamidopropylammoniumchlorid), 3 Gew.-% Cetylalkohol, 1,5 Gew.-% DC 939 (Amodimethicon, Trimethylcetylammoniumchlorid und nichtionisches Tensid der Formel C₁₃H₂₇-(OC₂H₄)₁₂-OH), 1 Gew.-% Cetyltrimethylammoniumchlorid, 6,5 Gew.-% Hydroxypropyl-Guar, 1 Gew.-% Glycerolstearat, 2 Gew.-% Polyvinylpyrrolidon, Konservierungsmittel und bis zu 100 Gew.-% Wasser aufweist, wobei das Fettmaterial aus Cetylalkohol, Stearylalkohol, Behenylalkohol und Gemischen davon ausgewählt ist und wobei das Fettmaterial zusammen mit dem kationischen Tensid und dem wässrigen Träger eine lamellare Gelphase bildet.

2. Zusammensetzung zum Konditionieren der Haare nach Anspruch 1,
wobei das kationische Tensid ein kationisches Tensid in Form von quaternärem Ammonium ist, das der folgenden allgemeinen Formel (I) entspricht:
[N(R¹)(R²)(R³)(R⁴)]⁺(X)⁻ (I)
worin R¹, R², R³ und R⁴ jeweils unabhängig voneinander ausgewählt sind aus (a) einem aliphatischen Rest mit 1 bis 22 Kohlenstoffatomen oder (b) einem aromatischen, Alkoxy-, Polyoxyalkylen-, Alkylamido-, Hydroxyalkyl-, Aryl- oder Alkylarylrest mit bis zu 22 Kohlenstoffatomen; und X ein salzbildendes Anion wie jene ist, die aus Halogen (z.B. Chlorid, Bromid), Acetat-, Citrat-, Lactat-, Glycolat-, Phosphat-, Nitrat-, Sulfat- und Alkylsulfatresten ausgewählt sind.

3. Zusammensetzung zum Konditionieren der Haare nach Anspruch 1,
wobei das kationische Tensid ein Salz eines Amins ist, das der folgenden allgemeinen Formel (II) entspricht:
R¹-C(O)-N(H)-R²-N(R³)(R⁴) (II)
worin R¹ eine Fettsäurekette ist, die 12 bis 22 Kohlenstoffatome enthält, R² eine Alkylengruppe ist, die 1 bis 4 Kohlenstoffatome enthält, und R³ und R⁴ unabhängig voneinander eine Alkylgruppe mit 1 bis 4 Kohlenstoffatomen sind.

4. Zusammensetzung zum Konditionieren der Haare nach einem der Ansprüche 1 bis 3,
wobei das Polygalatomannan mit hydrophilen und hydrophoben Substituenten ein Poly(alkylether) eines Polygalactomannans mit hydrophilen bzw. hydrophoben Alkylethersubstituenten ist.

5. Zusammensetzung zum Konditionieren der Haare nach Anspruch 4,
wobei der hydrophile Substituent die Hydroxypropylgruppe ist und der hydrophobe Substituent eine lineare Alkylgruppe mit 14 bis 28 Kohlenstoffatomen oder ein Gemisch solcher Alkyle ist.

## Revendications

1. Composition de conditionnement capillaire comprenant un tensioactif cationique, un matériau gras, un polygalactomannane présentant des substituants hydrophiles et hydrophobes, et un support aqueux, dans laquelle la composition n'est pas une composition de coiffage comprenant 1 % en poids de Styleze W20 (terpolymère de vinylpyrrolidone/diméthylaminopropylméthacrylamide et de chlorure de lauryldiméthylméthacrylamidopropylammonium), 3 % en poids d'alcool cétylique, 1,5 % en poids de DC 939 (amodiméthicone, chlorure de triméthylcétylammonium, et tensioactif non ionique de formule C₁₃H₂₇-(OC₂H₄)₁₂-OH), 1 % en poids de chlorure de cétyltriméthylammonium, 6,5 % en poids d'hydroxypropylguar, 1 % en poids de stéarate de glycérol, 2 % en poids de polyvinylpyrrolidone, des conservateurs et jusqu'à 100 % en poids d'eau, dans laquelle le matériau gras est choisi parmi l'alcool cétylique, l'alcool stéarylique, l'alcool béhénylique et des mélanges de ceux-ci, et dans laquelle le matériau gras avec le tensioactif cationique et le support aqueux, forme une phase de gel lamellaire.

2. Composition de conditionnement capillaire selon la revendication 1, dans laquelle le tensioactif cationique est un tensioactif cationique d'ammonium quaternaire correspondant à la formule générale (I) suivante :
[N(R¹)(R²)(R³)(R⁴)]⁺(X)⁻ (I)
dans laquelle R¹, R², R³, et R⁴ sont chacun indépendamment choisis parmi (a) un groupe aliphatique de 1 à 22 atomes de carbone, ou (b) un groupe aromatique, alcoxy, polyoxyalkylène, alkylamido, hydroxyalkyle, aryle ou alkylaryle ayant jusqu'à 22 atomes de carbone ; et X est un anion formant un sel tel que ceux choisis parmi des radicaux halogène, (par exemple chlorure, bromure), acétate, citrate, lactate, glycolate, phosphate nitrate, sulfate et alkylsulfate.

3. Composition de conditionnement capillaire selon la revendication 1, dans laquelle le tensioactif cationique est un sel d'une amine correspondant à la formule générale (II) suivante :
R¹-C(O)-N(H)-R²-N(R³)(R⁴) (II)
dans laquelle R¹, est une chaîne d'acide gras contenant de 12 à 22 atomes de carbone, R² est un groupe alkylène contenant de un à quatre atomes de carbone, et R³ et R⁴, sont indépendamment, un groupe alkyle ayant de un à quatre atomes de carbone.

4. Composition de conditionnement capillaire selon l'une quelconque des revendications 1 à 3, dans laquelle le polygalactomannane présentant des substituants hydrophiles et hydrophobes est un poly(alkyléther) d'un polygalactomannane présentant des substituants alkyléther hydrophiles et hydrophobes respectivement.

5. Composition de conditionnement capillaire selon la revendication 4, dans laquelle le substituant hydrophile est le groupe hydroxypropyle et le substituant hydrophobe est un groupe alkyle linéaire contenant de 14 à 28 atomes de carbone ou un mélange de tels groupes alkyle.
